(19) Europäisches Patentamt
European Patent Office
Office européen des brevets

(11) **EP 3 476 338 A1**

(12) **EUROPEAN PATENT APPLICATION**
published in accordance with Art. 153(4) EPC

(43) Date of publication:
**01.05.2019 Bulletin 2019/18**

(51) Int Cl.:
***A61B 17/34*** *(2006.01)*          ***A61B 1/00*** *(2006.01)*
***G02B 23/24*** *(2006.01)*

(21) Application number: **16907372.3**

(22) Date of filing: **30.11.2016**

(86) International application number:
**PCT/JP2016/085615**

(87) International publication number:
**WO 2018/003140 (04.01.2018 Gazette 2018/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **27.06.2016 JP 2016126708**

(71) Applicant: **A-Traction Inc.**
**Kashiwa-shi, Chiba 277-8577 (JP)**

(72) Inventors:
• **ANDO, Takehiro**
  **Chiba 277 8577 (JP)**
• **MIYAMOTO, Hiroyuki**
  **Chiba 277 8577 (JP)**

(74) Representative: **Müller-Boré & Partner**
**Patentanwälte PartG mbB**
**Friedenheimer Brücke 21**
**80639 München (DE)**

(54) **SURGICAL ASSISTANCE DEVICE, METHOD AND PROGRAM FOR CONTROLLING SAME, AND SURGICAL ASSISTANCE SYSTEM**

(57)    A surgery assisting apparatus comprises: distance measuring means for measuring a distance to an object in a body cavity; a hollow tube including a cylindrical portion to be partially inserted into the body cavity and a portion that performs distance measurement by the distance measuring means, the hollow tube enabling distance measurement by the distance measuring means in a given position on a circumference at a predetermined distance from a major axis of the cylindrical portion; and control means for controlling the position on the circumference around the major axis where the distance measuring means performs distance measurement, such that approach of a medical instrument inserted into the body cavity through the cylindrical portion to the object in the body cavity is sensed, wherein the control means controls the position on the circumference around the major axis in accordance with an advancing direction of a distal end of the medical instrument.

FIG. 1

**Description**

TECHNICAL FIELD

The present invention relates to a surgery assisting apparatus, a control method and program of the same, and a surgery assisting system.

BACKGROUND ART

Laparoscopic surgery is conventionally performed by forming a small-diameter hole in the abdominal wall and inserting a medical instrument such as an endoscope or therapeutic device into the body cavity from the small-diameter hole. Since the range observable by the endoscope in the body cavity is narrower than the range within which the distal end of the medical instrument operates, there is the possibility that a part of the medical instrument touches and damages an organ or the like outside the field of view of the endoscope. To prevent contact on an organ by a medical instrument like this, methods of sensing contact or approach of a medical instrument to an organ or the like are known (PTL 1 and PTL 2).

PTL 1 discloses a technique which uses an endoscope having joints in a body insertion portion and controls the posture of the body insertion portion by sensing contact with a peripheral organ or another medical instrument by using a contact sensor of each joint. On the other hand, a technique which prevents contact between a medical instrument and an organ by measuring the three-dimensional shape of the organ by using a stereoscopic camera inserted from the body wall is known. PTL 2 discloses a technique in which a stereoscopic camera is installed in the distal end portion of a trocar inserted into the body cavity and a noninterference region between a medical instrument and an organ or the like positioned in the body cavity is set based on the three-dimensional position of the organ obtained by using the stereoscopic camera.

CITATION LIST

PATENT LITERATURE

PTL 1: Japanese Patent Laid-Open No. 2004-81277
PTL 2: Japanese Patent Laid-Open No. 2015-159955

SUMMARY OF INVENTION

TECHNICAL PROBLEM

However, a medical instrument touches an organ in the technique disclosed in PTL 1, so this technique cannot be used for a fragile organ which is damaged only when touched. Also, in the technique disclosed in PTL 2, the camera is fixed in the distal end of the trocar for which only the angle of insertion into the abdominal cavity can be changed, so a dead angle is produced depending on the moving direction of a medical instrument. Consequently, the three-dimensional position of the medical instrument in a predetermined direction is not generated or the three-dimensional position is not the latest one in some cases. This sometimes makes it impossible to accurately sense approach of the medical instrument to a momentarily changing organ or the like. PTL 2 further discloses a method of setting a noninterference region by installing an electronic distance meter in the distal end of a medical instrument, instead of the stereoscopic camera. However, the measurement range is narrow because the electronic distance meter is installed in the distal end of a medical instrument, and it is impossible to detect interference in a shaft portion (a portion closer to the body wall than the distal end) other than the distal end of the medical instrument.

The present invention has been made in consideration of the above problems. That is, it is an object of the present invention to provide a surgery assisting apparatus capable of accurately sensing approach of a medical instrument inserted into the body cavity to an object in the body cavity, a control method and program of the same, and a surgery assisting system.

SOLUTION TO PROBLEM

To solve the above problem, for example, a surgery assisting apparatus of the present invention includes the following arrangement. That is, the surgery assisting apparatus is characterized by comprising distance measuring means for measuring a distance to an object in a body cavity, a hollow tube including a cylindrical portion to be partially inserted into the body cavity and a portion that performs distance measurement by the distance measuring means, the hollow tube enabling distance measurement by the distance measuring means in a given position on a circumference at a predetermined distance from a major axis of the cylindrical portion, and control means for controlling the position on the circumference around the major axis where the distance measuring means performs distance measurement, such that approach of a medical instrument inserted into the body cavity through the cylindrical portion to the object in the body cavity is sensed, wherein the control means controls the position on the circumference around the major axis in accordance with an advancing direction of a distal end of the medical instrument.

ADVANTAGEOUS EFFECTS OF INVENTION

The present invention makes it possible to accurately sense approach of a medical instrument inserted into the body cavity to an object in the body cavity.

Other features and advantages of the present invention will be apparent from the following description taken in conjunction with the accompanying drawings. Note that the same reference numerals denote the same or like components throughout the accompanying drawings.

BRIEF DESCRIPTION OF DRAWINGS

The accompanying drawings, which are incorporated in and constitute a part of the specification, illustrate embodiments of the invention and, together with the description, serve to explain the principles of the invention.

Fig. 1 is a view showing a configuration example of a surgery system including a surgery assisting apparatus according to an embodiment;
Fig. 2 is a view showing configuration examples of a hollow tube 6 and an optical part holder 5 according to the embodiment;
Fig. 3 is a view showing configuration examples of the hollow tube 6 and a hollow tube driving unit 7 according to the embodiment;
Fig. 4 is a view showing configuration examples of the hollow tube 6 and hollow tube driving unit 7 when using a hollow tube 41 according to the embodiment;
Fig. 5 is a view showing an example including a balloon 51 in the distal end of a mantle tube 4 according to the embodiment;
Fig. 6 is a view for explaining the optical axes of optical parts 21 and 22 according to the embodiment;
Fig. 7 is a view for explaining a method of controlling the sensing range of distance measurement in an object sensing process according to the embodiment;
Fig. 8 is a flowchart showing a series of operations of the object sensing process according to the embodiment;
Fig. 9 is a view showing the layout of optical parts according to another embodiment; and
Fig. 10 is a view showing the configuration of an optical part holder for sensing a stain according to still another embodiment.

DESCRIPTION OF EMBODIMENTS

Exemplary embodiments of the present invention will be explained in detail below with reference to the accompanying drawings.

(Configuration of Surgery Assisting Apparatus)

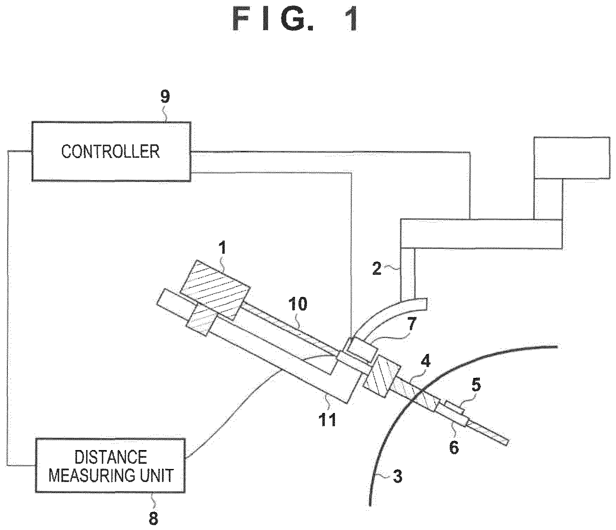

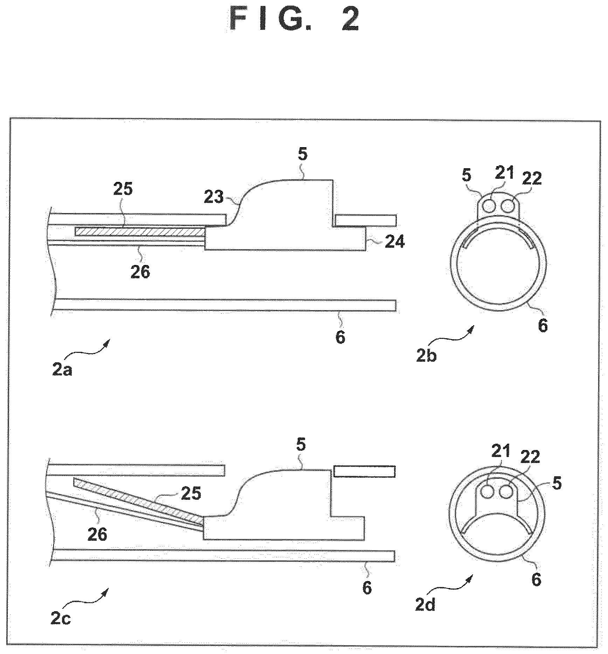

Fig. 1 is a view showing a functional configuration example of a surgery system including a surgery assisting apparatus according to an embodiment.
This surgery system includes a medical instrument 1, a medical instrument driving unit 2, a mantle tube (trocar) 4, an optical part holder 5, a hollow tube 6, a hollow tube driving unit 7, a distance measuring unit 8, and a controller 9. Of these components, the surgery assisting apparatus according to this embodiment includes the optical part holder 5, the hollow tube 6, the hollow tube driving unit 7, the distance measuring unit 8, and the controller 9.
The medical instrument 1 includes forceps, tweezers, an electric knife, a suction tube, an ultrasonic scalpel, a hemostatic device, a radiofrequency ablation device, an endoscope, a thoracoscope, and a laparoscope, all of which are inserted into the body cavity and used, and is an arbitrary instrument having a straight shaft 10 which can be inserted into the body cavity through the hollow tube 6. Also, the distal end of the medical instrument 1 can have a degree of freedom of bending, and a device for driving this bending portion can be included in the medical instrument or medical instrument driving unit.
The medical instrument driving unit 2 includes a driving unit for manipulating the position/posture of the medical instrument 1 from outside the body, and is configured so as to be able to control the position/posture of the medical instrument by at least two degrees of freedom. First, the medical instrument driving unit 2 can change the insertion angle of the medical instrument 1 with respect to the contact point between the mantle tube 4 and a body wall 3 (that is, a hole in the body wall 3). Also, the medical instrument driving unit 2 includes a rail 11 which is drivable parallel to the shaft 10 of the medical instrument, and can move the medical instrument 1 in the major-axis direction of the shaft 10. The mechanism of the driving unit can be a known mechanism, for example, a mechanism using an R guide, a mechanism using parallel links, or a mechanism using a vertical articulated arm, and is not limited to the mechanism shown in Fig. 1. Any of these driving units includes a plurality of positioning actuators such as servo motors, and current position information such as joint angles of the mechanism can be obtained from encoders included in the actuators. Accordingly, the distal end position of the medical instrument 1 can be known on the coordinate system of the medical instrument driving unit 2.
The mantle tube 4 has a hollow structure for inserting the medical instrument 1 or the like into it, and is inserted into a hole formed in the body wall 3 when in use. The mantle tube 4 according to this embodiment is configured so as to be connectable to the end portion, on the side of the body wall 3, of the rail 11 of the medical instrument driving unit 2. However, the mantle tube 4 can also be equivalent to a mantle tube used in ordinary laparoscopic surgery, provided that it is connectable to the medical instrument driving unit 2. Also, the inner diameter of this mantle tube is larger than the outer shape of the hollow tube 6 (to be described below) in order to insert the hollow tube 6.
In the vicinity of its distal end portion, the hollow tube 6 has the optical part holder 5 which can project outside the hollow tube 6, and is configured so as to be insertable into the mantle tube 4. Details of the configuration examples of the hollow tube 6 and optical part holder 5 will be explained with reference to Fig. 2. A hole having a predetermined size is formed in the circumferential surface close to the distal end portion of the hollow tube 6, and a portion of the optical part holder 5 is projectable outside the outer shape of the hollow tube 6 through the hole (2a in Fig. 2). Also, as indicated by 2b in Fig. 2, optical parts 21 and 22 for optical distance measurement are arranged in that portion of the optical part holder 5, which projects outside the outer shape of the hollow tube 6. These optical parts can include only optical fibers in order to reduce the weights or simplify the structures. Alternatively, each optical part can include one or more optical parts such as a lens, a diffraction grating, a mirror, a filter, a wavelength plate, a generation source (laser or LED), and a light-receiving portion (photodiode) in addition to the optical fiber, so as to improve the functionality implementable in the optical part holder 5.

With reference to 2a in Fig. 2 again, at least the root of the optical part holder 5 has a shape having a smooth inclined surface 23. The optical part holder 5 can also include a connecting portion 26 as a portion to be connected to the distance measuring unit 8. The connecting portion 26 is formed by an optical fiber or a cable for exchanging electrical signals, in accordance with the configuration of the optical part holder 5. The optical part holder 5 includes a stopper 24 for preventing projection more than necessary from the hollow tube 6. The optical part holder 5 is fixed to the hollow tube 6 by an elastic body 25. When no external force is applied to the optical part holder 5 from outside the hollow tube 6, the restoring force of the elastic body 25 causes the optical parts 21 and 22 of the optical part holder 5 to project from the outer diameter of the hollow tube 6. On the other hand, as indicated by 2c in Fig. 2, when an external force which pushes the optical part holder 5 into the hollow tube 6 is applied, the optical part holder 5 is accommodated in the hollow tube 6, and enters inside the outer diameter of the hollow tube 6. That is, as indicated by 2d in Fig. 2, the optical parts 21 and 22 of the optical part holder 5 are accommodated in the hollow tube 6.

When inserting the hollow tube 6 into the mantle tube 4, the user of the surgery assisting system pushes the optical part holder 5 into the hollow tube 6 as indicated by 2c or 2d in Fig. 2, and inserts the hollow tube 6 into the mantle tube 4. While the optical part holder 5 is passing inside the mantle tube 4, an external force from the mantle tube 4 acts on the optical part holder 5, so the optical part holder 5 maintains the state of 2c or 2d in Fig. 2. When the optical part holder 5 has passed through the mantle tube 4, the restoring force of the elastic body 25 causes the optical parts 21 and 22 to project outside the outer diameter of the hollow tube (that is, 2a or 2b). In this state, at least a space in which the shaft 10 of the medical instrument 1 can pass is secured in the hollow tube 6, so the shaft 10 of the medical instrument 1 can be inserted into the body cavity through the hollow tube 6. When the shaft 10 of the medical instrument 1 is inserted into the hollow tube 6, the optical part holder 5 projects outside the hollow tube 6 and is fixed in this state, thereby preventing fluctuations in positions of the optical parts 21 and 22 during distance measurement.

On the other hand, when removing the medical instrument 1, the medical instrument 1 is first pulled out from the hollow tube 6, and the hollow tube 6 is pulled out from the mantle tube 4. Since the shape of the root of the optical part holder 5 has the inclined surface 23 including a smooth inclined portion, the optical part holder 5 is pushed by the distal end portion of the mantle tube 4 and automatically accommodated in the hollow tube 6 (without a touch on the optical part holder 5 by the user). That is, when the optical part holder 5 comes in contact with the end portion of the mantle tube 4 when the hollow tube 6 is pulled out from the mantle tube 4, the inclined surface 23 generates a force of accommodating the optical part holder 5 in the cylindrical portion of the hollow tube 6. Note that the optical part installation surface of the optical part holder 5 and the top of the optical part holder 5 form a corner in the above-described example, but a smooth inclined surface may also be formed. This facilitates insertion of the hollow tube 6, and can also reduce the influence when the optical part holder 5 touches an organ or the like in the body cavity.

In this embodiment as described above, the optical parts 21 and 22 projecting from the circumferential surface of the hollow tube 6 perform distance measurement. This makes it possible to sense approach of not only the distal end of the medical instrument 1 but also a part of the shaft 10 to an object in the body cavity. In addition, the optical part holder 5 is accommodated in the hollow tube 6 when the hollow tube 6 is inserted into the mantle tube 4, and projected outside the hollow tube 6 when the hollow tube 6 has passed through the mantle tube 4. When inserting the optical part into the body cavity, therefore, it is unnecessary to form a new hole in the abdominal wall or enlarge the hole in the abdominal wall, and this can reduce the burden on the patient. Furthermore, the shape of the optical part holder 5 has the inclined surface 23. This obviates the need for a mechanism of electrical control or the like for accommodating the optical part holder 5 in the hollow tube 6, and makes it possible to simplify the structure of the hollow tube 6 and secure a wider space in the hollow tube 6.

Note that in the example indicated by 2a and 2c in Fig. 2, the hole for projecting the optical part holder 5 is formed in the circumferential surface of the hollow tube 6. This can prevent a liquid or the like sticking to the shaft 10 from directly sticking to the optical part holder 5 when the medical instrument 1 moves in the removing direction. On the other hand, instead of forming the hole, it is also possible to make a cut extending from the end face of the hollow tube 6, or form the optical part holder 5 at the distal end of the hollow tube 6.

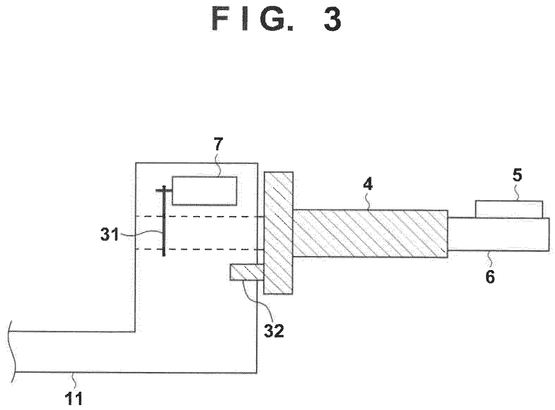

As shown in Fig. 3, the hollow tube 6 is fixed to a support portion of the rail 11 of the medical instrument driving unit 2 in a state in which the hollow portion 6 has only a degree of freedom of rotation around the major axis. As a method of fixing the hollow tube 6, a bearing structure using a general bearing or the like can be used. The major-axis direction of the hollow tube 6 is parallel to the major-axis direction of the shaft 10 of the medical instrument 1 and to the direction of the rail 11 of the medical instrument driving unit 2. This allows the shaft 10 of the medical instrument 1 to freely move forward and backward in the hollow tube 6.

The hollow tube driving unit 7 includes a driving unit for rotating the hollow tube 6 around the axis, and is fixed to the support portion of the rail 11 of the medical instrument driving unit 2. The hollow tube driving unit 7 has a driving mechanism 31 capable of rotating the support portion of the rail 11 and the hollow tube 6 relative to each other. As the driving mechanism 31, it is possible to use general torque transmitting mechanisms such as a gear, a belt pulley, and a friction wheel, and examples are not limited to them. An attaching/detaching mechanism 32 detachably fixes the mantle tube 4 and the support portion of the rail 11, thereby restricting at least movement in the major-axis direction. The attaching/detaching mechanism 32 need only be a mechanism capable of temporary fixation in the major-axis direction, such as a fitting mechanism, a magnet, or an adhesive material, and can have any structure.

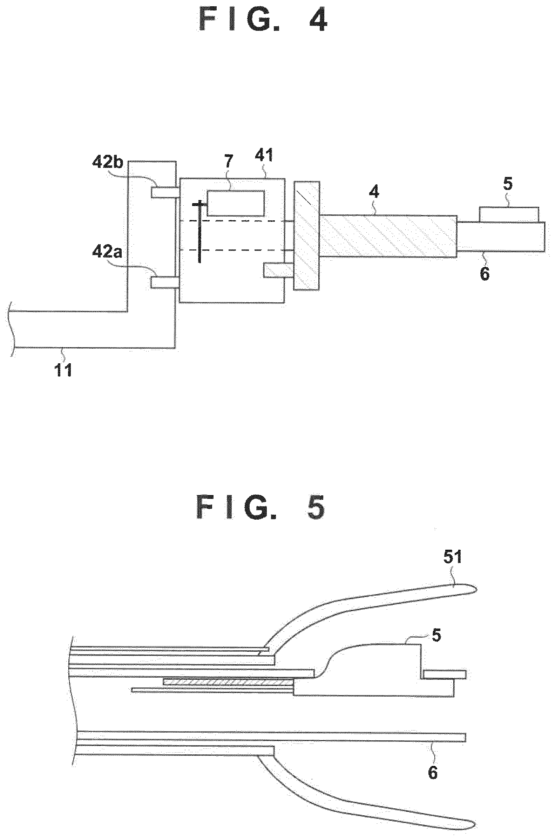

As shown in Fig. 4, the hollow tube driving unit 7 may also be included in a detachable hollow tube 41. In this case, the hollow tube driving unit 7 and driving mechanism 31 are integrally detached from the support portion of the rail 11. Attaching/detaching mechanisms 42a and 42b of the hollow tube 41 fix the hollow tube 41 so as to restrict rotation around the major axis and movement in the major-axis direction of the hollow tube 41 with respect to the support portion of the rail 11. Like the attaching/detaching mechanism 32 of the mantle tube 4, each of the attaching/detaching mechanisms 42a and 42b can be formed by a fitting mechanism, a magnet, an adhesive material, or the like. By thus making the hollow tube 41 detachable, it is possible to reduce the complicatedness when installing the surgery assisting apparatus or surgery assisting system.

The distal end shape of the mantle tube 4 can be a known shape used in ordinary laparoscopic surgery, or a shape including a balloon 51 surrounding the optical part holder 5 shown in Fig. 5. The use of the balloon 51 makes it possible to prevent a body fluid dropping from the root of the mantle tube 4 from sticking to the optical part holder 5. Accordingly, it is possible to prevent a decrease in distance measurement accuracy caused by a liquid or the like sticking to the optical part holder 5.

The distance measuring unit 8 implements a distance measuring means for measuring the distance to an object in the body cavity by emitted light, together with the optical parts 21 and 22 arranged in the optical part holder. To implement this function, it is possible to use the principles of known optical range finders, such as a method of estimating a distance by measuring the time required for light to go and return, a method of estimating a distance by using the interference of light, a method of estimating a distance by the intensity of reflected light, and a method of estimating a distance by triangulation. Generally, a method of measuring the distance to an object in the body cavity by using emitted light can perform distance measurement simpler and stabler than that performed by a method using a stereoscopic image.

In the example shown in Fig. 1, the distance measuring unit 8 includes a measuring unit accommodating a light generation source, a light-receiving portion, and an arithmetic chip for estimating the distance, and this measuring unit is connected to the optical parts 21 and 22 (that is, there is a distance between the optical part holder 5 and distance measuring unit 8). The measuring unit can include one or more optical parts such as a lens, a diffraction grating, a mirror, a filter, a wavelength plate, generation source (laser or LED), and a light-receiving portion (photodiode) in accordance with the members included in the optical part holder 5. Instead of the above-described example, the measuring unit can be incorporated into the periphery of the medical instrument driving unit 2 or hollow tube driving unit 7, and can also be incorporated into the optical part holder 5.

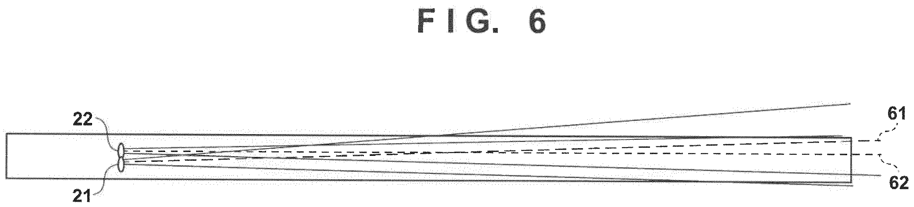

The optical part 22 on the exit side of the optical part holder 5 has a function of collimating light, so an object is irradiated with spot light. The spot diameter on the object is equivalent to the diameter of the shaft of a medical instrument to be inserted, and is 20 mm or less. The optical part 21 on the light-receiving side of the optical part holder 5 receives the reflected light of light emitted from the optical part 22. As shown in Fig. 6, an optical axis 61 of the optical part 21 on the light-receiving side is slightly inclined toward the optical part 22 on the exit side, and so adjusted as to be able of efficiently obtain the reflected light of light emitted on an object. Note that in Fig. 6, the dotted lines indicate the optical axes of the optical parts, and the solid lines indicate the exit range of the optical part 22 and the light-receiving range of the optical part 21. Note that the optical part 22 on the light exit side is basically parallel to the major-axis direction of the shaft 10 of the medical instrument 1, but can also have an angle at which the optical part 22 separates from the major-axis direction of the shaft. In this case, the optical axis 61 on the light-receiving side similarly has an angle at which the optical axis 61 separates from the shaft.

Since the hollow tube 6 can be rotated by the above-described hollow tube driving unit 7, the distance measurement range extends to the region of a cylindrical shape or conical shape surrounding the shaft 10 of the medical instrument 1, in addition to the distance measurement range when the optical part holder 5 is standing still. In this embodiment, the origin of distance measurement is the position of the optical part 22. However, the present invention is not limited to this, and the origin of distance measurement can be an arbitrary position as long as the relative positions of the optical part 22 and medical instrument driving unit 2 are fixed.

The controller 9 includes a central arithmetic device such as a CPU or MPU, a ROM, and a RAM, and executes software stored in the ROM or a recording medium (not shown), thereby controlling an object sensing process (to be described later). The controller 9 is connected to the medical instrument driving unit 2, the hollow tube driving unit 7, and the distance measuring unit 8. For example, the controller 9 obtains current position information for specifying the distal end position of the medical instrument, rotation information for specifying the rotation of the hollow tube 6, and distance information for specifying the distance of the distance measuring unit 8, from the joint angles of the medical instrument driving unit 2 and the position of the rail 11, and performs an arithmetic operation. Also, based on the result of this arithmetic operation, the controller 9 transmits control information for controlling these units. Furthermore, the controller 9 is connected to an output unit (not shown) including a liquid crystal panel, a speaker, or a vibrating member, and outputs sounds, images, characters, or vibrations to the user as needed.

(Series of Operations According to Object Sensing Process)

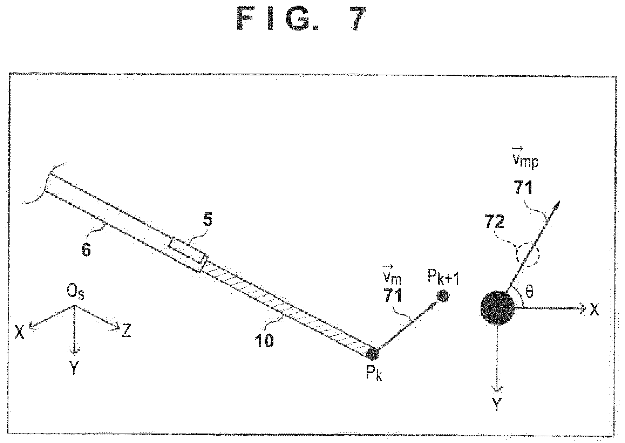

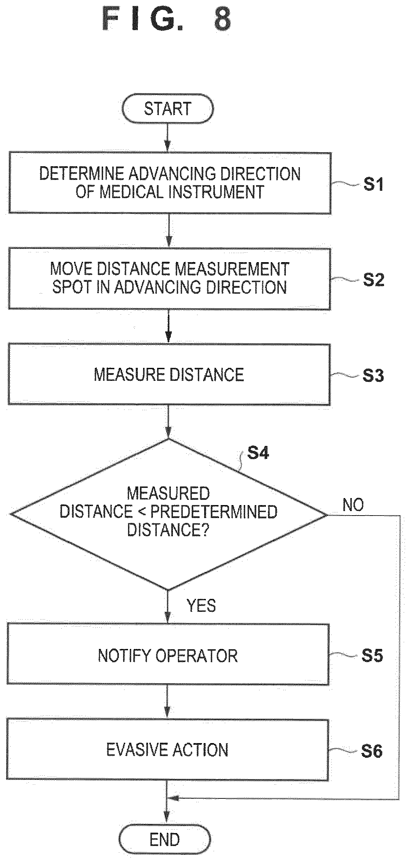

Next, a series of operations according to the object sensing process of the surgery assisting apparatus according to this embodiment will be explained with reference to Figs. 7 and 8. This process is started when, for example, the controller 9 has received an instruction to move the distal end of the shaft 10 of the medical instrument 1. Note that the controller 9 implements this process by controlling each unit by executing a program stored in the ROM.

In step S1, the controller 9 determines the advancing direction of the medical instrument 1. More specifically, when moving the medical instrument 1 in the body cavity by the medical instrument driving unit 2, as shown in the left view of Fig. 7, the advancing direction of the distal end of the medical instrument 1 can be represented by a three-dimensional vector extending from the distal end of the medical instrument 1. For example, the controller 9 first specifies the distal end position of the shaft 10 of the medical instrument 1 based on the current position information obtained from the medical instrument driving unit 2. More specifically, assume an orthogonal coordinate system $O_s$ having an arbitrary axis parallel to the shaft 10 of the medical instrument 1. Two remaining axes can freely be determined as long as mutual conversion from the coordinate system of the medical instrument driving unit 2 is possible. For example, when a command angle from the hollow tube driving unit 7 is 0°, it is possible to select a vector parallel to the direction from the shaft 10 of the medical instrument 1 to the optical part, and define the orthogonal coordinate system Os by using the vector. For the sake of simplicity, assume that the Z-axis is the direction of the shaft 10 of the medical instrument 1, and the X-axis is the direction when the command angle from the hollow tube driving unit 7 is 0°. Assume also that the optical axes for distance measurement intersect each other on the X-axis when the command angle of the hollow tube driving unit 7 is 0°. Note that the coordinate system determination method is not limited to the above method as long as mutual conversion to the coordinate system of the medical instrument driving unit 2 is possible.

The distal end position of the shaft 10 of the medical instrument 1 is known from the current position information obtained from the medical instrument driving unit 2, and the hollow tube 6 is fixed to the rail 11 of the medical instrument driving unit 2. Therefore, the controller 9 can specify the distal end position and three-dimensional vector of the shaft 10 in relation to the origin of distance measurement (the position of the optical part 22) fixed to the hollow tube 6. Likewise, the controller 9 can also specify the distal end position of the shaft 10 to be moved at the next time. The coordinates of these distal end positions are represented by:

$$P_k = (x_k, y_k, z_k)$$

$$P_{k+1} = (x_{k+1}, y_{k+1}, z_{k+1})$$

By using these distal end positions, the controller 9 obtains a vector $V_m$ extending from a point at a given time to a point at the next time. That is, this vector is represented by:

$$\vec{v}_m = P_{k+1} - P_k$$

The controller 9 thus calculates a three-dimensional vector representing the advancing direction of the distal end position of the shaft 10 of the medical instrument 1, based on an instruction to move the distal end of the shaft 10 to a predetermined position (the controller 9 may also set this predetermined position).

In step S2, the controller 9 rotates the hollow tube 6 by controlling the hollow tube driving unit 7, such that a distance measurement spot as a distance measurement sensing range 72 exists in the moving direction of the distal end of the shaft 10. This is equivalent to rotating the hollow tube 6 such that a direction obtained by projecting a three-dimensional vector 71 onto a plane perpendicular to the major axis of the hollow tube 6 matches a direction from the major axis of the hollow tube 6 to the optical part 22. More specifically, based on the three-dimensional vector 71, the controller 9 rotates the hollow tube 6 by driving the hollow tube driving unit 7, so that the three-dimensional vector 71 and the distance measurement sensing range 72 (that is, the optical axis on the exit side) intersect each other (the right view in Fig. 7). For example, the above-described vector Vm is projected onto an XY plane of the orthogonal coordinate system Os. That is, it is only necessary to calculate the inner product of basic vectors representing the X-axis and Y-axis as follows:

$$\vec{v}_{mp} = (\vec{v}_m \cdot \vec{i}_s, \vec{v}_m \cdot \vec{j}_s)$$

where vectors is and $j_s$ are the basic vectors representing the X-axis and Y-axis of the orthogonal coordinate system Os. The advancing direction of the distal end position of the shaft 10 of the medical instrument 1 is represented by a vector $V_{mp}$ on the XY plane of the orthogonal coordinate system Os, and hence can be represented as an angle $\theta$ to the X-axis by assuming a polar coordinate system around the Z-axis. As described above, the X-axis is the direction in which the command angle of the hollow tube driving unit is 0°. Therefore, the optical axis for distance measurement and the three-dimensional vector representing the advancing direction can be crossed by directly inputting the angle $\theta$ as the command angle of the hollow tube driving unit 7. Consequently, the optical part holder 5 faces the space in which the distal end of the shaft 10 of the medical instrument 1 moves, and the distance measurement sensing range 72 exists in this space. Note that another method may also be used if it is possible to rotate the hollow tube 6 so that the distance measurement sensing range 72 exists in the advancing direction of the distal end position of the shaft 10.

In step S3, the controller 9 instructs the distance measuring unit 8 to measure the distance while moving the distal end position of the shaft 10 of the medical instrument 1 in the advancing direction.

In step S4, the controller 9 determines whether the medical instrument 1 has approached an object in the body cavity by taking account of the distance obtained from the distance measuring unit 8 and the distal end position of the shaft 10 of the medical instrument 1. More specifically, the controller 9 determines whether the distance measured by the distance measuring unit 8 is shorter than a predetermined threshold. If the measured distance is shorter than the predetermined threshold, the controller 9 determines that the object is sensed, and advances the process to step S5. As the predetermined threshold, it is possible to use, for example, a value obtained by adding a predetermined margin to the distance from the intersection of a perpendicular line, which is drawn from the origin of distance measurement to the major axis of the shaft 10, to the distal end position of the shaft 10. By thus moving the optical part holder 5 in the advancing direction of the distal end position of the shaft 10, it is possible to sense approach of the shaft 10 (including the distal end position) of the medical instrument to the object. This makes it possible to accurately sense approach of the medical instrument inserted into the body cavity to the object in the body cavity. On the other hand, if the measured distance is equal to or larger than the predetermined threshold, no object is sensed. Accordingly, the controller 9 returns the process to step S3 in order to continue the distance measurement.

In step S5, the controller 9 performs notification to the user. In this notification to the user, the controller 9 causes the output unit (not shown) to output information indicating the existence of the object in the body cavity by, for example, a sound, light, or a vibration.

In step S6, the controller 9 takes evasive action by controlling the movement of the distal end position of the shaft 10 of the medical instrument 1. As this evasive action, the controller 9 can execute one of, for example, stopping the movement at once, pulling out the medical instrument 1 from the body so as to obtain a sufficient distance from the object position, and changing the advancing direction to a direction in which no object exists. It is also possible to perform one of the notification to the user in step S5 and the evasive action in step S6. When performing the both, the notification to the user may also be performed after the evasive action. After that, the controller 9 terminates the series of operations.

In this embodiment as explained above, the optical part holder 5 in which the optical part 22 included in the distance measuring unit 8 is placed can be accommodated in the hollow tube 6, and the optical part 22 of the optical part holder 5 projects outside the hollow tube 6 when a part of the hollow tube 6 is inserted into the body cavity. Also, approach of the shaft 10 (including the distal end position) of the medical instrument 1 to an object is sensed by rotating the optical part 22 in the advancing direction of the distal end position of the shaft 10 of the medical instrument 1. This makes it possible to accurately sense approach of the medical instrument inserted into the body cavity to an object in the body cavity.

Note that the present invention is not limited to the above-described embodiment, and can include other embodiments without departing from the spirit and scope of the invention.

<Other Embodiments>

For example, an example using optical distance measurement as the optical parts 21 and 22 has been explained in the above-described embodiment. However, a stereoscopic camera may also be formed by using a camera as each of the optical parts 21 and 22. Alternatively, it is also possible to use both optical distance measurement and a stereoscopic camera. When performing three-dimensional measurement in the body cavity by using the stereoscopic camera, optical distance measurement can be used if the accuracy of feature point extraction decreases and this decreases the accuracy of distance measurement.

In the above-described embodiment, an example in which distance measurement is performed by projecting the optical parts of the optical part holder 5 from the circumferential surface of the hollow tube 6 has been explained. However, it is also possible to form a gap between the hollow tube 6 and the section of the medical instrument 1 by increasing the diameter of the hollow tube 6, and perform distance measurement by arranging the optical parts 21 and 22 in this gap. That is, it is also possible to perform distance measurement around the medical instrument 1 by arranging the optical parts inside the circumferential surface of the hollow tube 6.

In the above-described embodiment, an example in which the pair of optical parts 21 and 22 are projected from the circumferential surface of the hollow tube 6 and the hollow tube 6 is rotated in accordance with the movement of the medical instrument 1 has been explained. However, it is also possible to arrange a plurality of pairs (for example, four or eight pairs) of optical parts outside (or inside) the hollow tube 6 along the outer circumference, and select a pair of optical parts for performing distance measurement in accordance with the movement of the medical instrument 1. Distance measurement in the advancing direction of the medical instrument inserted into the body cavity can be performed in this case as well.

The surgery assisting apparatus according to this embodiment has been explained by taking, as an example, the case in which the apparatus includes the optical part holder 5, the hollow tube 6, the hollow tube driving unit 7, the distance measuring unit 8, and the controller 9. However, the surgery assisting apparatus can further include an arbitrary component included in the surgery assisting system in addition to these components. In the above-described embodiment, an example in which the optical parts of the optical part holder 5 are projected from the hollow tube 6 by the elastic body 25 of the hollow tube 6 has been explained. However, the optical parts of the optical part holder 5 may also be projected from the hollow tube 6 in accordance with the insertion of the medical instrument 1 into the hollow tube 6 without using any elastic body.

Furthermore, the components of the above-described surgery assisting system may also be implemented as separated components or an integrated component. In addition to the case in which the controller reads out a program of a computer for executing the above-described processing from the storage medium and executes the program, the present invention can include a case in which the program is obtained by wired communication or wireless communication and executed.

In the above-described embodiment, an example in which the optical parts accommodated in the optical part holder 5 are the pair of the optical part 21 on the light-receiving side and the optical part 22 on the exit side has been explained. However, the above-described optical part holder 5 may also include a plurality of optical parts on one of the exit side and light-receiving side, instead of including one optical part on each side.

For example, the optical part holder 5 includes one optical part 21 on the light-receiving side, and a plurality of optical parts 22 on the exit side. In this configuration, the optical axes of the plurality of exit-side optical parts 22 incline to each other (are not parallel), and point in different directions. Furthermore, each of the optical axes of the plurality of exit-side optical parts 22 may point in a direction different from that of the optical axis of the light-receiving-side optical part 21.

When the optical part holder includes the plurality of exit-side optical parts 22, for example, the distance measuring unit 8 switches (selects) every predetermined time interval the optical parts 22 for emitting light of the plurality of exit-side optical parts 22, thereby sequentially emitting exit light in a plurality of directions. The distance measuring unit 8 may also be able to switch the optical parts 22 for emitting light in accordance with the advancing direction of the distal end position of the shaft 10 of the medical instrument 1 or the rotating direction of the hollow tube 6, thereby measuring the distance to a region closer to the advancing direction or rotating direction. By measuring the distance by switching the directions of exit light, it is possible to sense obstacles in a broader range without moving the optical part itself. The distance measuring unit 8 includes a plurality of light sources such as laser diodes, and switches the optical parts 22 for emitting light by electrically switching the light sources for generating light. The optical parts 22 for emitting light may also be switched by using one light source such as a laser diode, and a mechanism such as an optical switch for switching optical paths between the light source and the plurality of optical parts 22.

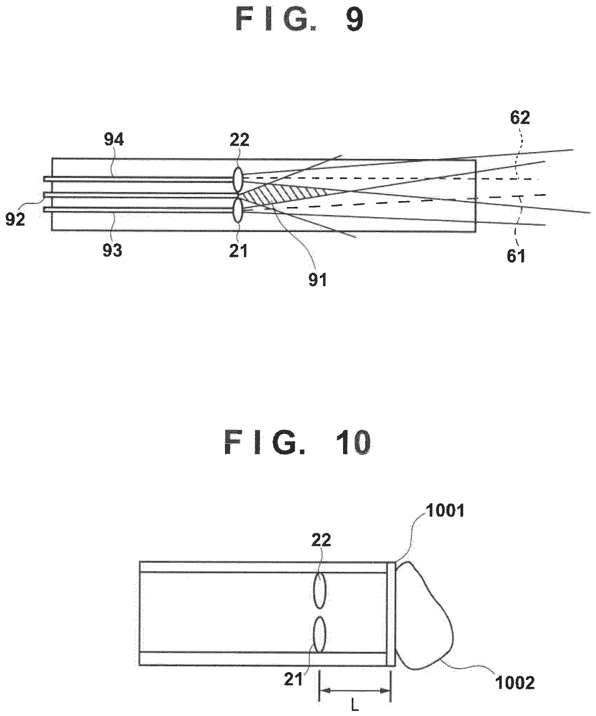

On the other hand, the optical part holder 5 may also include one exit-side optical part 22, and a plurality of light-receiving-side optical parts 21. The plurality of light-receiving-side optical parts 21 make distance measurement at higher sensitivity possible. More specifically, a region where the emitted light beam and the light beam to be received do not intersect each other and the sensitivity extremely decreases exists near the light-receiving-side optical part 21 and light-emitting-side optical part 22 shown in Fig. 6. Contrary to this, a new optical fiber is placed as the light-receiving-side optical part 21 near the light-receiving-side optical part 21 and light-emitting-side optical part 22. For example, as shown in Fig. 9, a new light-receiving-side optical part (optical fiber 92) is placed between the light-receiving-side optical part 21 and light-emitting-side optical part 22, thereby forming a new measurement region 91. Note that optical fibers 93 and 94 respectively represent a fiber for transmitting light from the light-receiving-side optical part 21, and a fiber for transmitting light to the exit-side optical part 22. As described above, however, the optical parts 21 and 22 may also simply be formed by the distal ends of the optical fibers 93 and 94. By thus further placing the light-receiving-side optical part (optical fiber 92) near the optical parts, it is possible to broaden the measurement region and further stabilize measurement. Note that it is desirable to use an optical fiber having as large a numerical aperture (NA) as possible as the optical fiber 92. In the example shown in Fig. 9, the NA of the optical fiber 92 is larger than that of the other light-receiving optical fiber. Note that when a light-receiving-side optical part is added, the distance measuring unit 8 sometimes requires a new light-receiving part. However, the distance measuring unit 8 may also use only one light-receiving part together with the prepared light-receiving-side optical fiber 93.

In the above-described embodiment, an example in which the optical parts of the optical part holder 5 are exposed in the body cavity has been explained. Since the optical part holder 5 is used in the body cavity, a substance (a sticking substance 1002) in the body cavity such as a blood may stick to the optical part and cause a detection error of the distance. To prevent this, as shown in Fig. 10, the optical parts 21 and 22 can be sealed by setting a glass plate 1001 in front of the optical parts 21 and 22, so that a body fluid and the like do not touch the optical parts. In this case, the optical parts 21 and 22 can be arranged in positions retracted by a predetermined distance L from the distal end of that portion of the optical part holder 5, which projects from the hollow tube 6. The distance L is equal to or longer than a shortest distance measurable by the optical parts 21 and 22. When the optical part holder 5 is thus configured, if a substance sticks to the surface of the glass plate 1001, this sticking substance is sensed as an obstacle at the distance L, and this makes stain sensing possible.

If the controller 9 determines that a stain is sensed because the distance obtained from the distance measuring unit 8 is the distance L, the controller 9 notifies the user that the stain is sensed. In addition to this notification, the controller 9 can further encourage the user to perform cleaning, and can also automatically perform a process of cleaning the optical part holder 5. As a method of automatically cleaning the optical part holder 5, it is possible to use a known method of cleaning the glass plate 1001 by spraying a gas or liquid from an additionally prepared nozzle placed near the glass plate 1001.

The present invention is not limited to the above-described embodiments, and various changes and modifications can be made within the spirit and scope of the present invention. Therefore, to apprise the public of the scope of the present invention, the following claims are made.

This application claims the benefit of Japanese Patent Application No. 2016-126708, filed June 27, 2016, which is hereby incorporated by reference herein in its entirety.

**Claims**

1. A surgery assisting apparatus **characterized by** comprising:

   distance measuring means for measuring a distance to an object in a body cavity;
   a hollow tube including a cylindrical portion to be partially inserted into the body cavity and a portion that performs distance measurement by the distance measuring means, the hollow tube enabling distance measurement by the distance measuring means in a given position on a circumference at a predetermined distance from a major axis of the cylindrical portion; and
   control means for controlling the position on the circumference around the major axis where the distance measuring means performs distance measurement, such that approach of a medical instrument inserted into the body cavity through the cylindrical portion to the object in the body cavity is sensed,
   wherein the control means controls the position on the circumference around the major axis in accordance with an advancing direction of a distal end of the medical instrument.

2. The surgery assisting apparatus according to claim 1, **characterized by** further comprising:

   hollow tube driving means for rotating the cylindrical portion of the hollow tube around the major axis with respect to the medical instrument,
   wherein the control means controls the position on the circumference around the major axis where the distance measuring means performs distance measurement, by controlling the hollow tube driving means.

3. The surgery assisting apparatus according to claim 1 or 2, **characterized in that** the control means controls the position on the circumference around the major axis where the distance measuring means performs distance measurement, such that the distance measuring means performs distance measurement in the advancing direction of the distal end of the medical instrument.

4. The surgery assisting apparatus according to any one of claims 1 to 3, **characterized in that** the control means controls the position on the circumference around the major axis where the distance measuring means performs distance measurement, such that the advancing direction of the distal end of the medical instrument intersects a direction of an optical axis of the distance measuring means.

5. The surgery assisting apparatus according to any one of claims 1 to 4, **characterized in that** in a case where the distance to the object in the body cavity measured by the distance measuring means is shorter than a predetermined distance taking account of a length to the distal end of the medical instrument, the control means senses approach of the medical instrument to the object in the body cavity.

6. The surgery assisting apparatus according to any one of claims 1 to 5, **characterized in that** the distance measuring means measures the distance to the object in the body cavity by using emitted light.

7. The surgery assisting apparatus according to any one of claims 1 to 6, **characterized in that**
the hollow tube further includes a holder including an optical part that performs distance measurement by the distance measuring means, the holder being configured such that the optical part projects outside a circumferential surface of the cylindrical portion or is accommodated in the cylindrical portion, and
in a case where the distance measuring means performs distance measurement, the optical part of the holder projects outside the circumferential surface of the cylindrical portion.

8. The surgery assisting apparatus according to claim 7, **characterized in that** the hollow tube is configured to enable the medical instrument to be inserted into the cylindrical portion, in a case where the optical part included in the holder projects outside the circumferential surface of the cylindrical portion.

9. The surgery assisting apparatus according to claim 7 or 8, **characterized in that**
a part of the cylindrical portion of the hollow tube is inserted into the body cavity through a mantle tube, and
the optical part included in the holder and accommodated in the cylindrical portion projects outside the circumferential surface of the cylindrical portion, in a case where the holder passes through the mantle tube.

10. The surgery assisting apparatus according to claim 9, **characterized in that** the holder includes an inclined portion which generates a force for accommodating the holder in the cylindrical portion, when the inclined portion comes in contact with an end portion of the mantle tube in a case where the hollow tube is pulled out from the mantle tube.

11. The surgery assisting apparatus according to any one of claims 7 to 10, **characterized in that**
the hollow tube further includes an elastic body which projects the optical part included in the holder outside the circumferential surface of the cylindrical portion, and
the elastic body is configured to accommodate the holder in the cylindrical portion in a case where the holder receives an external force from outside the cylindrical portion.

12. The surgery assisting apparatus according to any one of claims 1 to 11, **characterized in that** the advancing direction of the distal end of the medical instrument is produced by changing an insertion angle of the medical instrument and changing an insertion depth of the medical instrument.

13. The surgery assisting apparatus according to claim 1, **characterized in that**
a plurality of optical parts for performing distance measurement by the distance measuring means are arranged on the circumference at the predetermined distance from the major axis of the cylindrical portion of the hollow tube, and
the control means controls the position on the circumference around the major axis where the distance measuring means performs distance measurement, by selecting one or more of the plurality of optical parts.

14. A surgery assisting apparatus **characterized by** comprising:

distance measuring means for measuring a distance to an object in a body cavity by using an optical part which emits light; and
a hollow tube including a cylindrical portion to be partially inserted into the body cavity, and a holder in which a portion which causes the distance measuring means to measure a distance in a major-axis direction of the cylindrical portion can project outside the cylindrical portion,
wherein the hollow tube rotates around a major axis of the cylindrical portion such that approach of a medical instrument inserted into the body cavity through the cylindrical portion to the object in the body cavity is sensed, and
the distance measuring means measures the distance to the object in the body cavity by selecting one of a plurality of optical parts arranged in the holder such that optical-axis directions are different from each other.

15. A surgery assisting apparatus **characterized by** comprising:

distance measuring means for measuring a distance to an object in a body cavity by using an optical part which emits light; and
a hollow tube including a cylindrical portion to be partially inserted into the body cavity, and a holder in which a portion which causes the distance measuring means to measure a distance in a major-axis direction of the cylindrical portion can project outside the cylindrical portion,
wherein the hollow tube rotates around a major axis of the cylindrical portion such that approach of a medical instrument inserted into the body cavity through the cylindrical portion to the object in the body cavity is sensed, and

the holder further includes the optical part, and a wall which prevents a substance in the body cavity from sticking to the optical part, the optical part and the wall being spaced apart by a distance longer than a first distance at which the optical part can perform distance measurement such that sticking of the substance to the wall can be detected.

16. A surgery assisting system comprising:

a surgery assisting apparatus according to any one of claims 1 to 13; and

medical instrument driving means for controlling movement, in a body cavity, of the medical instrument inserted into the body cavity through the cylindrical portion, based on control information of the control means,

**characterized in that** in a case where approach of the medical instrument to the object in the body cavity is sensed, the control means controls the medical instrument driving means such that the medical instrument does not touch the object in the body cavity.

17. A method of controlling a surgery assisting apparatus including a distance measuring device for measuring a distance to an object in a body cavity, a hollow tube including a cylindrical portion to be partially inserted into the body cavity and a portion for performing distance measurement by the distance measuring device, the hollow tube enabling distance measurement by the distance measuring device in a given position on a circumference at a predetermined distance from a major axis of the cylindrical portion, and a control device, **characterized by** comprising:

a step of causing the control device to control the position on the circumference around the major axis where the distance measuring device performs distance measurement, such that approach of a medical instrument inserted into the body cavity through the cylindrical portion to the object in the body cavity is sensed,

wherein in the control step, the position on the circumference around the major axis is controlled in accordance with an advancing direction of a distal end of the medical instrument.

18. A program for causing a computer to execute the step of the method of controlling a surgery assisting apparatus according to claim 17.

19. A surgery assisting apparatus comprising:

distance measuring means for measuring a distance to an object in a body cavity; and

a hollow tube having a cylindrical portion to be partially inserted into the body cavity, the hollow tube enabling distance measurement by the distance measuring means in a given position around a major axis of the cylindrical portion, **characterized in that** the hollow tube further includes a holder in which a portion for performing distance measurement by the distance measuring means can project outside the cylindrical portion, and

the portion has an inclined portion in a position where the inclined portion comes in contact with another instrument into which the hollow tube is inserted in a case where the hollow tube is pulled out from the body cavity.

# F I G.  1

CONTROLLER 9

DISTANCE MEASURING UNIT 8

1

10

11

2

7

4

5

6

3

# F I G. 2

# F I G. 3

# F I G. 4

# F I G. 5

# FIG. 6

# F I G. 7

# F I G. 8

```
            ┌─────────┐
            │  START  │
            └─────────┘
                 │
                 ▼
   ┌──────────────────────────────────┐
   │   DETERMINE ADVANCING DIRECTION   │ ～ S1
   │      OF MEDICAL INSTRUMENT        │
   └──────────────────────────────────┘
                 │
                 ▼
   ┌──────────────────────────────────┐
   │     MOVE DISTANCE MEASUREMENT     │ ～ S2
   │     SPOT IN ADVANCING DIRECTION   │
   └──────────────────────────────────┘
                 │
                 ▼
   ┌──────────────────────────────────┐
   │         MEASURE DISTANCE          │ ～ S3
   └──────────────────────────────────┘
                 │
                 ▼
              S4
           ╱        ╲
          ╱ MEASURED  ╲       NO
      ╱ DISTANCE < PREDETERMINED ╲──────┐
          ╲  DISTANCE? ╱                │
           ╲        ╱                   │
              │ YES                     │
              ▼                         │
   ┌──────────────────────────────────┐│
   │         NOTIFY OPERATOR           │ ～ S5
   └──────────────────────────────────┘│
                 │                      │
                 ▼                      │
   ┌──────────────────────────────────┐│
   │         EVASIVE ACTION            │ ～ S6
   └──────────────────────────────────┘│
                 │                      │
                 ▼◄─────────────────────┘
            ┌─────────┐
            │   END   │
            └─────────┘
```

# FIG. 9

# FIG. 10

## INTERNATIONAL SEARCH REPORT

| | International application No. |
|---|---|
| | PCT/JP2016/085615 |

**A.   CLASSIFICATION OF SUBJECT MATTER**
*A61B17/34*(2006.01)i, *A61B1/00*(2006.01)i, *G02B23/24*(2006.01)i

According to International Patent Classification (IPC) or to both national classification and IPC

**B.   FIELDS SEARCHED**

Minimum documentation searched (classification system followed by classification symbols)
A61B17/34, A61B1/00, G02B23/24

Documentation searched other than minimum documentation to the extent that such documents are included in the fields searched
Jitsuyo Shinan Koho        1922-1996   Jitsuyo Shinan Toroku Koho   1996-2017
Kokai Jitsuyo Shinan Koho   1971-2017   Toroku Jitsuyo Shinan Koho   1994-2017

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
|---|---|---|
| A | JP 2015-159955 A  (Olympus Corp.),<br>07 September 2015 (07.09.2015),<br>paragraphs [0022] to [0060]; fig. 1 to 7<br>& US 2016/0354164 A1<br>paragraphs [0013] to [0088]; fig. 1 to 7<br>& WO 2015/129834 A1      & EP 3111878 A1 | 1-19 |
| A | JP 2014-132980 A  (Advanced Healthcare Co.,<br>Ltd.),<br>24 July 2014 (24.07.2014),<br>paragraphs [0022] to [0056]; fig. 1 to 6<br>& US 2014/0194732 A1<br>paragraphs [0033] to [0074]; fig. 1 to 6 | 1-19 |

| ☒  Further documents are listed in the continuation of Box C. | ☐  See patent family annex. |
|---|---|

\*     Special categories of cited documents:
"A"   document defining the general state of the art which is not considered     to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search<br>    05 April 2017 (05.04.17) | Date of mailing of the international search report<br>    18 April 2017 (18.04.17) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2016/085615

C (Continuation). DOCUMENTS CONSIDERED TO BE RELEVANT

| Category* | Citation of document, with indication, where appropriate, of the relevant passages | Relevant to claim No. |
|---|---|---|
| A | JP 2012-78709 A (Olympus Corp.),<br>19 April 2012 (19.04.2012),<br>paragraphs [0052] to [0059]; fig. 10 to 12<br>(Family: none) | 1-19 |
| A | JP 2016-16053 A (Kyocera Optec Co., Ltd.),<br>01 February 2016 (01.02.2016),<br>paragraphs [0023] to [0026]; fig. 4<br>(Family: none) | 19 |
| E,X | JP 6108509 B1 (A-Traction Inc.),<br>05 April 2017 (05.04.2017),<br>claims 1 to 17; entire text; all drawings<br>(Family: none) | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004081277 A
- JP 2015159955 A
- JP 2016126708 A